# FASCICULE DE BREVET EUROPEEN

(11) **EP 1 280 481 B1**
(45) Date de publication et mention de la délivrance du brevet: **26.07.2006**
(21) Numéro de dépôt: 01934094.2
(22) Date de dépôt: 11.05.2001
(51) Int. Cl.: A61F 2/44

(54) **IMPLANT INTERSOMATIQUE ANTERIEUR LOMBAIRE**
ANTERIORES LUMBALES ZWISCHENKÖRPERIMPLANTAT
ANTERIOR LUMBAR INTERBODY IMPLANT

(30) Priorité: 11.05.2000 FR 0006023
(43) Date de publication de la demande: 05.02.2003
(73) Titulaire: SCIENT'X, 78284 Guyancourt (FR)
(72) Inventeur: LEMAIRE, Jean-Philippe, F-21910 Saulon La Chapelle (FR)
(74) Mandataire: Thibault, Jean-Marc
(86) Numéro de dépôt international: PCT/FR2001/001431
(87) Numéro de publication internationale: WO 2001/085069

(56) Documents cités:
- WO-A-00/19941
- WO-A-97/23175
- WO-A-98/34568
- WO-A-99/56676

## Description

La présente invention concerne un implant intersomatique destiné à être inséré dans l'espace intervertébral après excision discale, défini entre deux vertèbres adjacentes en vue de restituer une hauteur convenable intervertébrale et d'assurer une fusion osseuse entre lesdites vertèbres adjacentes.

L'objet de l'invention vise, plus précisément, un implant intersomatique du type lombaire destiné à être logé dans un espace intervertébral après excision discale, défini entre deux vertèbres lombaires adjacentes.

Dans l'état de la technique, il est connu d'insérer un implant intersomarique dans l'espace intervertébral défini entre deux vertèbres lombaires adjacentes. De nombreuses formes de réalisation de tels implants ont été proposées dans l'art antérieur. Par exemple, il est connu, par le document WO 97/23 175 un implant intersomatique lombaire se présentant sous la forme d'une cage comportant deux parois sagittales reliées entre elles par une paroi transversale antérieure et par une paroi transversale postérieure. Les parois délimitent entre elles un volume ouvert adapté pour recevoir un produit de comblement osseux appelé greffon osseux, destiné à venir en contact avec l'os spongieux des plateaux vertébraux, permettant de favoriser la fusion osseuse entre les deux vertèbres. Cet implant comporte, également, au moins deux parois faisant saillie par rapport à ses faces supérieure et inférieure en étant conformées pour s'insérer progressivement dans l'os spongieux lors de l'introduction et ensuite pour immobiliser l'implant.

Il doit être considéré qu'un greffon osseux placé à l'intérieur d'un tel implant laisse généralement substituer des parties qui sont saillantes par rapport aux faces transversales de l'implant. Or, lors de l'implantation de la cage dans l'espace intervertébral, il se produit un frottement entre l'implant et les vertèbres conduisant à une détérioration du greffon osseux pour les parties situées au niveau des faces transversales de l'implant. Il s'ensuit qu'un tel greffon osseux ne se trouve pas en contact avec l'os spongieux des plateaux vertébraux des vertèbres après l'implantation de la cage.

La demande de brevet WO 00/19941 décrit un implant réalisé en un matériau résorbable et présentant deux faces principales munies de crans d'ancrage. De même, la demande de brevet WO 95/56676 décrit un implant délimitant un logement de réception pour un greffon osseux. Un tel implant est équipé d'une pointe d'ancrage dans les vertèbres.

L'objet de l'invention vise à remedier aux inconvénients énoncés ci-dessus en proposant un implant intersomatique destiné à être inséré dans l'espace intervertébral défini entre deux vertèbres lombaires voisines, conçu pour permettre, par l'intermédiaire d'un greffon osseux placé dans l'implant, d'assurer une bonne continuité osseuse entre l'os spongieux des plateaux vertébraux des deux vertèbres lombaires voisines.

Pour atteindre cet objectif, l'implant intersomatique est conforme à la revendication 1.

Diverses autres caractéristiques ressortent de la description faite ci-dessous en référence aux dessins annexés qui montrent, à titre d'exemples non limitatifs, des formes de réalisation et de mise en oeuvre de l'objet de l'invention.
La **fig. 1** est une vue en perspective d'un exemple de réalisation d'un implant conforme à l'invention.
La **fig. 2** est une vue sagittale d'un implant prise sensiblement selon la flèche **F**_{**2**} de la **fig. 1.**
La **fig. 3** est une vue transversale d'un implant illustré aux **fig. 1** et **2.**
Les **fig. 4** et **5** sont des vues sagittales montrant l'implant selon l'invention, respectivement dans une position en cours d'implantation et dans une position finale d'implantation permettant d'expliciter les avantages de l'invention.

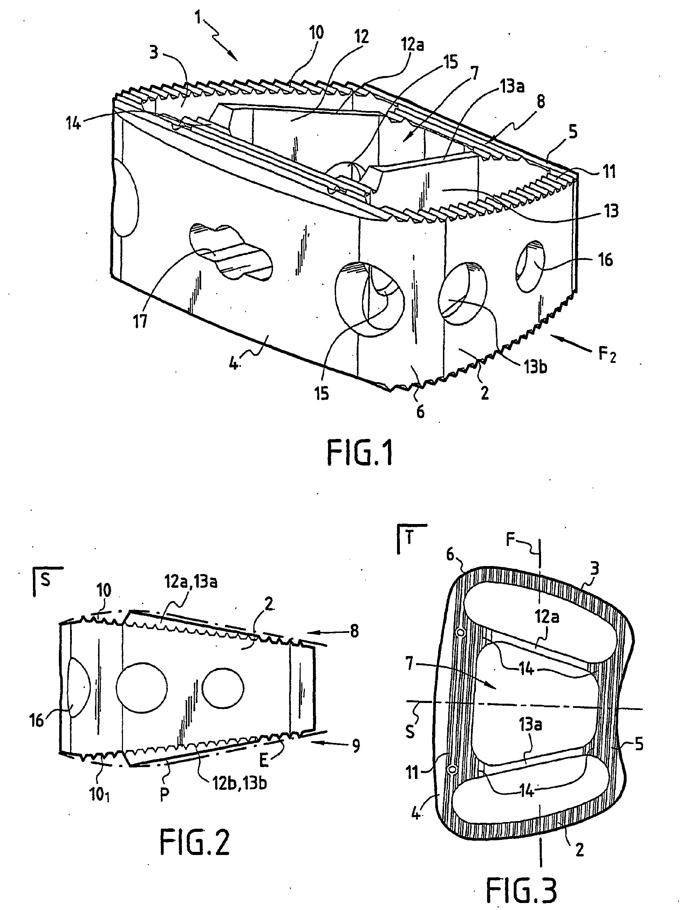

Tel que cela ressort plus précisément des **fig. 1** à **3**, l'implant intersomatique selon l'invention se présente sous la forme d'une cage **1** de forme générale parallélépipèdique destinée à être insérée dans l'espace intervertébral après excision discale, défini entre deux vertèbres lombaires adjacentes et désigné dans la suite de la description par espace intervertébral **E.** La cage **1** comporte une première paroi sagittale **2** et une seconde paroi sagittale 3 reliées entre elles par une paroi transversale dite antérieure **4** et par une paroi transversale dite postérieure **5.** Dans l'exemple illustré, les parois sagittales **2, 3** s'élèvent perpendiculairement à un plan transversal **T** et s'étendent de manière inclinée par rapport à un plan **S,** dit sagittal ou antéro-postérieur perpendiculaire au plan transversal **T.** Plus précisément, les parois sagittales **2, 3** convergent entre elles à partir de la paroi antérieure **4.** Les parois transversales antérieure **4** et postérieure **5** s'étendent sensiblement parallèlement l'une à l'autre et à un plan frontal **F** perpendiculaire au plan sagittal **S**.

De préférence, la cage **1** comporte des congés de raccordement **6** aménagés entre les parois sagittales **2, 3** et les parois transversales **4, 5** selon leur face verticale externe permettant de disposer d'une cage **1** avec des coins arrondis extérieurement. Tel que cela ressort plus précisément de la **fig. 3,** chaque paroi sagittale **2** et **3,** ainsi que la paroi transversale antérieure **4,** possèdent dans le plan transversal **T** perpendiculaire au plan frontal **F,** une courbure convexe, tandis que la paroi transversale postérieure **5** possède une courbure concave pour laisser dégager le canal vertébral.

La cage **1** présente intérieurement un volume **7** délimité par les faces verticales internes des parois **2** à **5** et destiné à être rempli par un produit de comblement osseux appelé greffon osseux adapté à la fusion intersomatique. Ce volume **7** s'ouvre selon une première face transversale **8,** dite supérieure dans l'exemple illustré, et une deuxième face transversale **9,** dite inférieure dans l'exemple considéré. Les parois **2** à **5** présentent d'un côté, des rebords **10** délimitant la face transversale supérieure **8** et, de l'autre côté, des rebords **10**_{**1**} délimitant la face transversale inférieure **9.**

La cage **1** comporte des crans **11** aménagés sur les rebords **10, 10**_{**1**} des parois **2** à **5** pour permettre un accrochage de la cage sur les vertèbres sus et sous-jacentes. Les crans **11** s'étendent parallèlement les uns des autres et par rapport au plan frontal **F.** D'une manière générale, il doit être compris que les faces transversales supérieure **8** et inférieure **9** correspondent à l'enveloppe passant par le sommet des crans **11.**

Conformément à l'invention, la cage **1** comporte au moins une et, dans l'exemple illustré, quatre rampes **12a, 12b, 13a, 13b** s'étendant chacune entre les parois transversales antérieure **4** et postérieure **5** et adaptée, conçue ou conformée pour assurer une fonction d'écartement des vertèbres, comme cela apparaîtra dans la suite de la description. Chaque rampe d'écartement **12a - 13a et 12b 13b** s'étend de manière saillante par rapport à une face transversale voisine, respectivement **8** et **9.** Chaque rampe d'écartement **12a, 12b, 13a, 13b** possède par rapport à la face transversale voisine **8, 9,** un profil qui augmente progressivement en direction de la paroi transversale antérieure **4.** Dans l'exemple illustré, les rampes d'écartement **12a, 12b, 13a, 13b** s'étendent perpendiculairement par rapport au plan transversal **T** en étant inclinées par rapport au plan sagittal **S.** Plus précisément, les rampes d'écartement **12a, 13a,** d'une part, et **12b, 13b,** d'autre part, convergent entre elles à partir de la paroi antérieure **4** en étant symétriques par rapport au plan sagittal **S.**

Les rampes d'écartement **12a, 12b** et **13a, 13b** s'étendent en saillie par rapport aux faces sagittales **8, 9** qui convergent entre elles en direction de la paroi transversale postérieure **5,** de sorte que la cage **1** possède une section tronconique dans le plan sagittal **S** permettant de définir un angle de rétablissement de lordose. Le choix de la hauteur des parois transversales antérieure **4** et postérieure **5** permet de disposer d'une gamme de cages avec des angles de lordose de rétablissement de valeur différente.

Selon une caractéristique avantageuse de réalisation, chaque rampe d'écartement **12a, 12b, 13a, 13b** est raccordée à chaque paroi transversale **4, 5** par une zone de liaison **14** prolongeant la paroi transversale au même niveau que la partie correspondante des parois sagittales **2, 3.** Il doit être compris que le profil de ces zones de liaison **14** suit le profil des parois sagittales **2, 3** situées en correspondance. Ces zones de liaison **14** présentent des rebords munis également de crans **11.** Il doit être ainsi considéré que les rampes d'écartement **12a, 12b, 13a, 13b** possèdent une longueur inférieure à la longueur des parois sagittales **2, 3.**

Selon une autre caractéristique avantageuse de réalisation, les rampes **12a, 12b, 13a, 13b** présentent un profil **P,** qui se trouve circonscrit à l'intérieur de l'espace intervertébral **E,** schématisé par des traits ininterrompus à la **fig. 2** et dans lequel l'implant est destiné à être placé. Il doit être compris que ces rampes d'écartement qui font saillie par rapport aux faces transversales **8** et **9** de l'implant, sont dimensionnées pour rester dans l'espace intervertébral **E** afin de ne pas pénétrer dans les vertèbres sus et sous-jacentes.

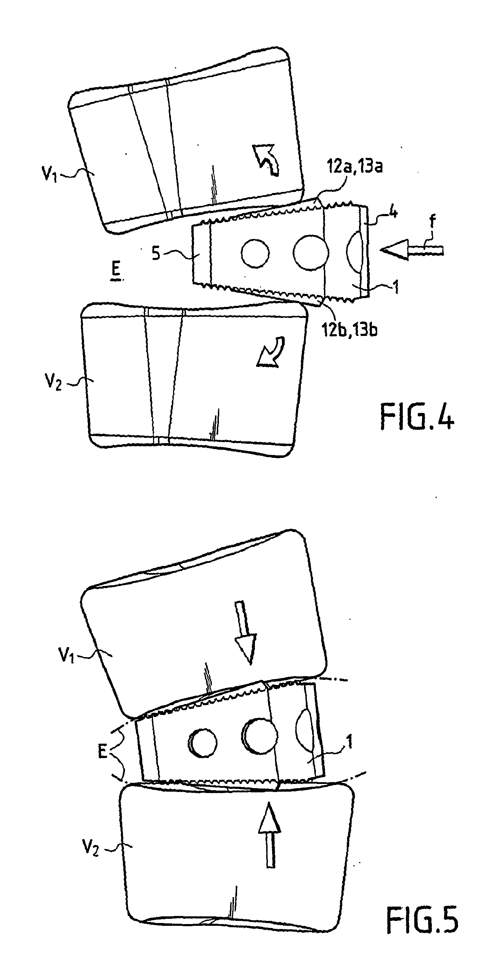

Les **fig. 4** et **5** illustrent de manière explicite les fonctions des rampes d'écartement de la cage **1** selon l'invention.

La cage **1** est destinée à assurer la restauration de l'espace intervertébral **E** entre deux vertèbres lombaires **V**_{**1**}**, V**_{**2**} représentées schématiquement. Tel que cela apparaît à la **fig. 4,** lors de la mise en place de l'implant par une voie d'abord antérieure, la cage **1** est déplacée selon une direction d'introduction **f** s'établissant dans le plan sagittal **S**. Lors de l'introduction de la cage **1** dans l'espace intervertébral **E,** avec la paroi transversale postérieure **5** située en aval selon le sens d'introduction, les rampes d'écartement **12a, 12b, 13a, 13b** permettent d'assurer une distraction ou un écartement relatif entre les vertèbres sus **V**_{**1**} et sous-jacente **V**_{**2**}**.** La présence de telles rampes d'écartement permet d'éviter le raclage par les vertèbres sus **V**_{**1**} et sous-jacente **V**_{**2**}**,** du greffon osseux placé dans le volume **7** de la cage. Compte tenu du profil des rampes d'écartement **12a, 12b, 13a, 13b** qui augmente de la paroi transversale postérieure **5** à la paroi transversale antérieure **4,** il peut être obtenu un écartement progressif des deux vertèbres **V**_{**1**}**, V**_{**2**}**.** Lorsque la cage **1** occupe sa position définitive dans l'espace intervertébral **E** (**fig. 5**), il apparaît que les rampes d'écartement **12a, 12b, 13a, 13b** restent confinées à l'intérieur de l'espace intervertébral E défini entre les vertèbres sus et sous-jacentes. Le maintien de la cage **1** entre les vertèbres sus et sous-jacente est assuré par les rebords des parois **2** à **5** qui se trouvent en appui sur les plateaux vertébraux. Compte tenu du fait que le greffon osseux placé à l'intérieur de la cage **1** n'a pas été expulsé en tout ou partie du volume 7 lors de la mise en place de l'implant, il peut être obtenu un bon contact entre le greffon osseux et l'os spongieux des plateaux vertébraux des vertèbres **V**_{**1**}**, V**_{**2**}**.**

Dans l'exemple de réalisation illustré, la cage **1** comporte quatre rampes d'écartement **12a, 12b, 13a, 13b.** Il doit être considéré que les rampes d'écartement **12a, 12b** définissent ensemble une première paroi intermédiaire **12** s'étendant selon un même plan incliné par rapport au plan sagittal **S.** De même, les rampes d'écartement **13a, 13b** définissent une deuxième paroi intermédiaire **13** s'étendant selon un même plan incliné par rapport au plan sagittal **S.** De préférence, ces parois intermédiaires **12** et **13** s'étendent de manière symétrique par rapport au plan sagittal **S** de l'implant.

Les parois intermédiaires **12, 13** divisent ainsi le volume de remplissage **7** en trois volumes élémentaires. De préférence, ces parois intermédiaires **12, 13** comportent des trous **15** permettant d'assurer une communication entre les différents volumes élémentaires définis entre elles. De préférence, les parois sagittales **2** et **3** et la paroi transversale antérieure **4** comportent également des trous de passage **16** favorisant la vascularisation du greffon osseux. Il est à noter que la paroi transversale antérieure **4** présente une lumière **17** de passage pour un outil assurant le compactage du greffon osseux placé à l'intérieur du volume 7.

Dans l'exemple illustré, la cage **1** comporte quatre rampes d'écartement **12a, 12b, 13a, 13b.** Bien entendu, il peut être envisagé de réaliser une cage **1** comportant une unique rampe d'écartement. Il peut être aussi envisagé de réaliser une cage comportant deux rampes d'écartement s'étendant selon un même plan.

L'invention n'est pas limitée aux exemples décrits et représentés, car diverses modifications peuvent y être apportées sans sortir de son cadre.

## Revendications

1. Implant intersomatique destiné à être inséré dans l'espace intervertébral **(E)** défini entre deux vertèbres lombaires voisines, dites sus **(V**_{**1**}**)** et sous-jacente **(V**_{**2**}**),** en vue du rétablissement anatomique de l'espace intervertébral, l'implant se présentant sous là forme d'une cage comportant au moins deux parois sagittales **(2, 3)** reliées entre elles par au moins des parois transversales antérieure **(4) et** postérieure **(5)** sensiblement parallèles à un plan frontal, les parois délimitant entre elles un volume **(7)** ouvert pour un remplissage osseux et présentant des rebords s'étendant d'un côté pour délimiter une première face transversale **(8)** et de l'autre côté, pour délimiter une deuxième face transversale **(9),** au moins une paroi s'étendant entre les parois transversales antérieure (**4**) et postérieure **(5)**, de manière saillante par rapport à une face transversale **(8, 9)**
**caractérisé en ce que** chaque paroi saillante est une rampe d'écartement **(12a, 12b, 13a, 13b)** conformée pour assurer un écartement relatif entre les vertèbres sus et sous-jacentes lors de l'introduction de l'implant dans l'espace intervertébral, et présentant un profil **(P)** qui se trouve circonscrit à l'intérieur de l'espace intervertébral **(E)** dans lequel l'implant est destiné à être placé afin de ne pas pénétrer dans les vertèbres sus et sous-jacentes - et **en ce que** les rebords des parois sagittales **(2, 3)** et transversales **(4, 5)** comportent des crans **(11)** s'étendant parallèlement au plan frontal **(F).**

2. Implant selon la revendication 1, **caractérisé en ce qu'**il comporte deux rampes d'écartement **(12a, 12b)** définissant une première paroi intermédiaire **(12)** s'étendant selon un même plan, chaque rampe d'écartement étant saillante par rapport à une face transversale **(8, 9).**

3. Implant selon la revendication 2, **caractérisé en ce qu'**il comporte une deuxième paroi intermédiaire **(13)** s'étendant de manière symétrique par rapport à l'autre paroi, cette deuxième paroi intermédiaire **(13)** étant munie de deux rampes , d'écartement **(13a, 13b)** s'étendant chacune de manière saillante par rapport à une face transversale **(8, 9).**

4. Implant selon l'une des revendications 1 à 3, **caractérisé en ce que** chaque rampe d'écartement **(12a, 12b, 13a, 13b)** possède par rapport à la face transversale voisine, un profil qui augmente progressivement en direction de la paroi transversale antérieure **(4).**

5. Implant selon l'une des revendications 1 à 4, **caractérisé en ce que** chaque rampe d'écartement **(12a, 12b, 13a, 13b)** est raccordée à une paroi transversale **(4, 5)** par une zone de liaison **(14)** prolongeant ladite paroi transversale au même niveau que les parois sagittales, de manière que chaque rampe d'écartement possède une longueur inférieure à la longueur d'une paroi sagittale **(2, 3).**

6. Implant selon l'une des revendications 1 à 3, **caractérisé en ce que** les parois sagittales **(2, 3)** et/ou les parois intermédiaires **(12, 13)** comportent des trous de passage **(15, 16).**

7. Implant selon les revendications 1 et 5, **caractérisé en ce que** les rebords des zones de liaison **(14)** comportent des crans **(11).**

8. Implant selon la revendication 2, **caractérisé en ce que** les rampes d'écartement **(12a, 12b, 13a, 13b)** s'étendent en saillie par rapport aux faces sagittales **(8, 9)** qui convergent entre elles en direction de la paroi transversale postérieure **(5),** de sorte que la cage possède une section tronconique dans le plan sagittal **(S),** en vue de définir un angle de rétablissement de la lordose.

9. Implant selon la revendication 1, **caractérisé en ce que** la paroi transversale postérieure **(5)** possède une courbure concave pour laisser dégager le canal vertébral.

10. Implant selon la revendication 1, **caractérisé en ce que** la paroi transversale antérieure **(4)** présente une lumière **(17)** de passage pour un outil assurant le compactage du remplissage osseux destiné à être placé à l'intérieur de l'implant.

## Claims

1. An intersomatic implant for insertion in the intervertebral space (E) defined between two adjacent lumbar vertebrae, referred to as an overlying vertebra (V₁) and an underlying vertebra (V₂), in order to reestablish the anatomical shape of the intervertebral space, the implant being in the form of a cage having at least two sagittal walls (2, 3) interconnected at least by an anterior transverse wall (4) and by a posterior transverse wall (5) that are substantially parallel to a front plane, the walls defining between them an open volume (7) for bone filling and presenting rims extending on one side to define a first transverse face (8) and on the other side to define a second transverse face (9), at least one wall extending between the anterior and posterior transverse walls (4, 5) so as to project relative to a transverse face (8, 9)
the implant being **characterised in that** each projecting wall is one spacer ramp (12a, 12b, 13a, 13b) shaped so as to determine relative spacing between the underlying and overlying vertebrae while the implant is being inserted into the intervertebral space and presenting a profile (P) which is to be found circumscribed inside the intervertebral space (E) in which the implant is to be placed so as to avoid penetrating into the underlying and overlying vertebrae and **in that** the rims of the sagittal and transverse walls (2, 3; 4, 5) include serrations (11) that extend parallel to the front plane (F).

2. An implant according to claim 1, **characterised in that** it has two spacer ramps (12a, 12b) defining a first intermediate wall (12) extending in a single plane, each spacer ramp projecting relative to a respective one of the transverse faces (8, 9).

3. An implant according to claim 2, **characterised in that** it has a second intermediate wall (13) extending symmetrically to the other wall, said second intermediate wall (13) being provided with two spacer ramps (13a, 13b) each projecting from a respective one of the transverse faces (8, 9).

4. An implant according to one of claims 1 to 3, **characterised in that** each spacer ramp (12a, 12b, 13a, 13b) possesses a profile relative to the adjacent transverse face which increases progressively going towards the anterior transverse wall (4).

5. An implant according to one of claims 1 to 4, **characterised in that** each spacer ramp (12a, 12b, 13a, 13b) is connected to a transverse wall (4, 5) by a connection zone (14) extending said transverse wall level with the sagittal walls so that each spacer ramp possesses a length that is shorter than the length of a sagittal wall (2, 3).

6. An implant according to one of claims 1 to 3, **characterised in that** the sagittal walls (2, 3) and/or the intermediate walls (12, 13) have through holes (15, 16).

7. An implant according to claims 1 and 5, **characterised in that** the rims of the connection zones (14) include serrations (11).

8. An implant according to claim 2, **characterised in that** the spacer ramps (12a, 12b, 13a, 13b) project relative to the sagittal faces (8, 9) which converge towards each other towards the posterior transverse wall (5) so that the cage is of frustoconical section in the sagittal plane (S) in order to define an angle for reestablishing lordosis.

9. An implant according to claim 1, **characterised in that** the posterior transverse wall (5) possesses concave curvature so as to leave the vertebral channel free.

10. An implant according to claim 1, **characterised in that** the anterior transverse wall (4) presents a slot (17) for passing a tool that serves to compact the bone filling that is to be placed inside the implant.

## Patentansprüche

1. Zwischenkörperimplantat, das dafür bestimmt ist, in den Zwischenwirbelraum (E), der zwischen zwei benachbarten lumbalen Wirbeln, die als Ober- (V1) und Unterwirbel (V2) bezeichnet werden, definiert ist, eingeführt zu werden, in Hinblick auf die anatomische Wiederherstellung des Zwischenwirbelraumes, wobei das Implantat die Form eines Käfigs aufweist, der mindestens zwei sagittale Wände (2, 3) umfasst, die miteinander durch mindestens eine anteriore (4) und eine posteriore (5) Querwand, die zu einer frontalen Ebene im wesentlichen parallel sind, verbunden sind, wobei die Wände zwischen einander ein Volumen (7) begrenzen, das für eine Knochenfüllung offen ist und das Kanten aufweist, die sich so erstrecken, dass sie an einer Seite eine erste Querseite (8) begrenzen und an der anderen Seite eine zweite Querseite (9) begrenzen, wobei sich mindestens eine Wand zwischen der anterioren (4) und der posterioren (5) Querwand hervorstehend in Bezug auf eine Querseite (8, 9) erstreckt, **dadurch gekennzeichnet, dass** jede hervorstehende Wand eine Rampe zur Beabstandung (12a, 12b, 13a, 13b) ist, die dafür geeignet ist, einen relativen Abstand zwischen den Ober- und Unterwirbeln bei der Einführung des Implantates in den Zwischenwirbelraum zu sichern und die ein Profil (P) aufweist, das innerhalb des Zwischenwirbelraumes (E), in dem das Implantat angeordnet werden soll, eingeschlossen ist, um nicht in die Ober- und Unterwirbel zu penetrieren und, dass die Kanten der sagittalen Wände (2, 3) und der Duerwände (4, 5) Einkerbungen (11) umfassen, die sich parallel zur frontalen Ebene (F) erstrecken.

2. Implantat nach Anspruch 1, **dadurch gekennzeichnet, dass** es zwei Rampen zur Beabstandung (12a, 12b) umfasst, die eine erste Zwischenwand (12) definieren, die sich gemäß einer selben Ebene erstreckt, wobei jede Rampe zur Beabstandung in Bezug auf eine Querseite hervorstehend ist.

3. Implantat nach Anspruch 2, **dadurch gekennzeichnet, dass** es eine zweite Zwischenwand (13) umfasst, die sich symmetrisch in Bezug auf die andere Wand erstreckt, wobei diese zweite Zwischenwand (13) mit zwei Rampen zur Beabstandung (13a, 13b) versehen ist, die sich hervorstehend in Bezug auf eine Querseite (8, 9) erstrecken.

4. Implantat nach einem der Ansprüche 1 bis 3, **dadurch gekennzeichnet, dass** jede Rampe zur Beabstandung (12a, 12b,13a, 13b) ein in Bezug auf die benachbarte Querseite progressiv steigendes Profil in der Richtung der anterioren Querwand (4) aufweist.

5. Implantat nach einem der Ansprüche 1 bis 4, **dadurch gekennzeichnet, dass** jede Rampe zur Beabstandung (12a, 12b, 13a, 13b) mit einer Querwand (4, 5) durch einen Verbindungsbereich (14), der die Querwand auf der selben Ebene wie die sagittalen Wände verlängert, derart verbunden ist, dass jede Rampe zur Beabstandung eine Länge aufweist, die kleiner ist als die Länge einer sagittalen Wand (2, 3).

6. Implantat nach einem der Ansprüche 1 bis 3, **dadurch gekennzeichnet, dass** die sagittalen Wände (2, 3) und / oder die Zwischenwände (12, 13) Durchgangslöcher (15, 16) umfassen.

7. Implantat nach einem der Ansprüche 1 bis 5, **dadurch gekennzeichnet, dass** die Kanten der Verbindungsbereiche (14) Einkerbungen (11) umfassen.

8. Implantat nach Anspruch 2, **dadurch gekennzeichnet, dass** die Rampen zur Beabstandung (12a, 12b, 13a, 13b) sich hervorstehend in Bezug auf die sagittalen Wände (8, 9), welche zueinander in Richtung der posterioren Querwand (5) konvergieren, derart erstrecken, dass der Käfig einen kegelstumpfförmigen Querschnitt in der sagittalen Ebene (S) aufweist, um einen Winkel zur Wiederherstellung der Lordose zu definieren.

9. Implantat nach Anspruch 1, **dadurch gekennzeichnet, dass** die posteriore Querwand (5) eine konkave Krümmung aufweist, um das Freilassen des Wirbelkanals zu veranlassen.

10. Implantat nach Anspruch 1, **dadurch gekennzeichnet, dass** die anteriore Querwand (4) einen Schlitz (17) zum Durchgang eines Werkzeugs aufweist, das die Verdichtung der Knochenfüllung sichert, die dafür bestimmt ist, innerhalb des Implantats angeordnet zu werden.
